# EUROPEAN PATENT APPLICATION

(11) **EP 1 393 746 A1**
(43) Date of publication of application: **03.03.2004**
(21) Application number: 02717170.1
(22) Date of filing: 16.04.2002
(51) Int. Cl.: A61K 39/395, A61P 31/04, A61P 31/12, C07K 16/12, C07K 16/08

(54) **HUMAN POLYCLONAL ANTIBODY COMPOSITIONS**

(30) Priority: 17.04.2001 JP 2001118610
(71) Applicant: Juridical Foundation, The Chemo-Sero-Therapeutic Research Institute, Kumamoto-shi, Kumamoto 860-8568 (JP); KIRIN BEER KABUSHIKI KAISHA, Tokyo 104-8288 (JP)
(72) Inventor: MAEDA, Hiroaki, Kumamoto-shi, Kumamoto 860-0076 (JP); UMEHASHI, Misako, Kumamoto-shi, Kumamoto 860-0084 (JP); NOZAKI, Chikateru, Kumamoto-shi, Kumamoto 862-8001 (JP)
(74) Representative: HOFFMANN - EITLE
(86) International application number: PCT/JP2002/003765
(87) International publication number: WO 2002/087615

(57) **Abstract**

A human polyclonal antibody having an antibacterial and/or antiviral activity with a desired titer and a wide spectrum; and human polyclonal antibody composition for preventing and treating infections which contain this antibody. The human polyclonal antibody composition for preventing or treating infections contain the human polyclonal antibody which is obtained by administering as immunogen a bacterium and/or a virus or a component of the bacterium and/or the virus to a non-human animal having a human antibody gene locus and has an antibody titer against the bacterium and/or the virus exceeding that of human pool plasma.

## Description

### TECHNICAL FIELD

The present invention relates to the field of medical drugs. Specifically, it relates to human polyclonal antibody composition having an antibacterial and or antiviral activity with a preferred titer and a wide spectrum for preventing or treating infections, namely diseases caused by bacteria or viruses and to the method for preparing the human polyclonal antibody compositions.

### BACKGROUND ART

In recent years, various kinds of higher medicinable antibiotic drugs have been developed, thereby medical treatments of infections have rapidly advanced. On the other hand, in the case of compromised hosts including patients with underlying diseases such as cancers, elder patients, and post-operative patients, the suppression of the immune system makes their infections severe, leading to difficulty in treatment even if mediciable antibiotic drugs are administered in many cases. The abuse of antibiotic drugs, however, induces drug-resistant bacteria including methicillin-resistant staphylococcus aureus (MRSA), penicillin-resistant pneumococcus, and drug-resistant pseudomonas aeruginosa, which reduce the effectiveness of antibiotic drugs. Against this background, infections remain ranked one of main causes of death.

As a treatment drug for severe infections, a human polyclonal antibody prepared from human plasma such as intravenous human immunoglobulin (hereafter, sometimes simply referred to as IVIG), namely a human immunoglobulin preparation has been administered, demonstrating its beneficial effect on these diseases (Journal of Japan Society of Chemotherapy, volume 48, p.199, 2000). Besides, IVIG is used in treating the patients infected by a certain kind of viruses such as cytomegalovirus(CMV) (The Latest Internal Medicine Taikei volume 26, Viral infections, edited by Hiroo IMURA et al, Nakayama, p.153, 1994). Additionally, to enhance the effects of treatment of and/or prevention against a certain kind of bacteria, bacterial toxin, or viruses, an attempt has been made to prepare an immunoglobulin drug, referred to as special immunoglobulin, from plasma with an antibody titer higher than that of any of these pathogenic immunogens. On the other hand, a very few of such a kind of drug products have been developed so far, for example antitetanic immunoglobulin and anti-HBs (hepatitis B surface immunoglobulin) immunoglobulin.

Although contributing not a little as a treatment for severe infections, IVIG have problems described below. Giving an example, IVIG prepared from human plasma (human pool plasma) pooled from an unspecified number of human individuals (on a scale of several thousands to 10 thousands) by ethanol fractionation contains an antibody with a wide spectrum against a wide variety of bacteria and viruses, though it may not yield an efficaciously satisfactory antibody titer because of being averaged. Furthermore, blended plasma is not always derived from the same donors, thereby the averaged antibody titer is not constant and over the long term, it may vary depending on the lot or manufacturer. Moreover, IVIG reflects the infection being going around at the time when it is manufactured, making it impossible to predict the antibody titer of a finished product. Thus, in the context of different preparation time and regional circumstances, the properties of the obtained antibodies, such as specificity, are not constant, leading to a difficulty in manufacturing products of uniformly high quality. This can be analogous to ready-made antibody drugs in the manufactured drugs have high versatility but do not address special requirements.

Even if the antibody drug with an average antibody titer as mentioned above is actually administered at high doses in treatment, in some cases, no effect of treatment can be expected because of its insufficient antibody titer. For example, in the case that in treating a patient with congenital hypogammaimmunoglobulinemia, who is infected by Pseudomonas aeruginosa, 200 to 500 mg/kg of immunoglobulin drug is first administered and 200 to 400 mg/kg of drug is added after 2 to 4 weeks after that, and finally to keep serum level of immunogammaglobulin (IgG) at about 400 to 500 mg/dl or more, his/her symptoms may not be alleviated in some cases (Today's meaning of Pseudomonas aeruginosa, edited by Atsushi SAITOU et al., Iyaku Journal, p.229, 1996).

In collecting human plasma as a material for immunoglobulin drugs, a screening is conducted to prevent any pathogens from accidentally mixing in, while no screening is generally conducted to discriminate human individuals with the antibody to a specific immunogen. For this reason, it is difficult to stably reserve a mass volume of human plasma with the high antibody titer against a specific immunogen, which in turn, makes it difficult practically to prepare the human polyclonal antibody specific to a desired immunogen. Meanwhile, it may be considered that material plasma for the antibody drug is obtained by immunizing with an immunogen, for example a vaccine, though generally, this method does not yield commercial drug products because a severe ethical problem arises.

To address the problem involved with IVIG mentioned above, a special immunoglobulin has been developed, a so-called custom-made which is prepared from the material plasma selected focusing on the antibody titer against a specific immunogen, thereby having a high antibody titer, though its application is actually confined to some cases because of problems involved with screening of material plasma and its limited volume. A few special immunoglobulin products have been developed including antitetanic immunoglobulin for treating the patients with tetanus and anti-HBs (hepatitis B S immunogen) immunoglobulin used for suppressing infection by Hepatitis B, which are relatively easy to obtain but applicable to only a limited number of patients.

The application of human monoclonal antibody (hereafter, sometimes referred to as MoAb) is also worth a consideration. At present, technologies developed for making mouse MoAb applicable to human patients besides the hybridoma approach, cloning of human antibodies by the phage display approach, and generating mice used for producing human antibodies have enabled the preparation of human MoAb with a desired immunogenic specificity, achieving various types of antibody drugs.

For MoAb, an antibody with a high titer can be selected by a screening using specific immunogen or pathogen. Generally, however, the MoAb can recognize only one epitope derived from one immunogen. Most of bacteria and viruses have many sub strains commonly called "serotype" therein and generally, the MoAb specific to one serotype does not react to other serotypes. Similarly, giving the MoAb to *Pseudomonas aeruginosa* as an example, the MoAb specific to a certain serotype (Today's meaning of Pseudomonas aeruginosa, edited by Atsushi SAITO et al., Iyaku Journal, p76, 1996) does not react to the serotypes other than serotype associated with it and therefore, a plenty of MoAbs had to be mixed into a cocktail (Today's meaning of *Pseudomonas aeruginosa*, edited by Atsushi SAITO et al., Iyaku Journal, p243, 1996; S.Sawada et al, J. Gen. Microbiol. vol.133,p.3581,1987).

Besides, since the surfaces of bacteria and viruses contain a plenty of neutralizing immunogens, the MoAb, which recognizes a certain neutralizing immunogen, does not always have full capacity of neutralization. Furthermore, most of the patients with common infections have infected by mixed kinds of other bacteria and/or viruses and for this reason, almost no effect can be expected when only MoAb, which recognizes one kind of serotype in one kind of pathogen, is administered. Thus, diseases for which the administration of only one kind of MoAb takes effects of treatment are few in any way in the clinical field of infections.

As mentioned above, the number of MoAbs to be mixed is limited, even if so done. As the number of MoAbs to be mixed increases, its preparation cost also rises and finally, may exceed the reasonable price for putting on the market as an antibody drug, thereby the mixture of MoAbs is not a realistic method. Actually, no human MoAb for treating infections has been made commercially available in Japan. For example, a plurality of pharmaceutical companies including U.S. Cutter, Sumitomo Pharmaceuticals (Today's meaning of *Pseudomonas aeruginosa*, edited by Atsushi SAITO et al., Iyaku Journal, p243, 1996), and Teijin (S.Sawada et al., J. Gen. Microbiol. vol.133,p.3581,1987) produced MoAbs to the O immunogen of *Pseudomonas aeruginosa* LPS for each serotype and then tried to mix 3 to 6 MoAbs to develop a cocktail drug. However, unfortunately, any of them has stopped their activities of development. Moreover, the MoAb to cytomegalovirus has been developed but not put on the market so far.

Thus, actually, it is under such a circumstance that no antibody drugs with the properties equivalent to those of polyclonal antibody, for example MoAb-based IVIG and special immunoglobulin, can be prepared. At present, especially in the case of infections, it cannot be expected that one kind of MoAb having effects of treatment on a wide range of infections is developed and only one antibody drug for treating infections is IVIG derived from human plasma.

### DISCLOSURE OF THE INVENTION

As mentioned above, at the present time, in the case of antibody drugs for treating infections, substantially only immunoglobulin drugs having a wide spectrum but with an average antibody titer are commonly used.

The objective of the invention is to overcome the problems involved not only with IVIG but also with special immunoglobulin and human MoAb drugs and provide an antibody drug having an antibody titer equivalent to that of a desired special immunoglobulin necessary for its associated immunogen, namely a titer equivalent to or higher than that of IVIG and a capacity of neutralization with a required range of spectrum.

The inventors of the invention focused attention on non-human animals having a human antibody gene locu.s and made an attempt to prepare a human polyclonal antibody having a high antibody titer against a bacterium and a virus with a wide range of antibody spectrum using the animals.

It is known that there are antibodies, which react to the pathogens or immunogens despite of being neither infected by a pathogen nor actively immunized (Dictionary of Immunology, TOKYO KAGAKU DOJIN, p.256, 1993), namely natural antibodies. These antibodies include anti-A antibody, anti-B antibody, and antibodies, which react to polysaccharide of a certain kind of bacterium. It is known that natural antibodies are characterized in that they have a wide spectrum of cross-reactivity despite of low ability. It may be assumed that some of these antibodies are produced under genetic dominance, namely under an environment in which immunogenic specificity has been set in a region V and the others have acquired cross-reactivity to immunogenic stimuli by bacteria such as intestinal bacteria (Medical Immunology, edited by Koukichi KIKUCHI, Nankodo, p.243, 1981; Immunological Illustrated (Original 5^{th} version) I. Roitt et al., Nankodo, p.166, 2000). The former are thought to contain sequences, which have selected, stored in the bodies, and acquired in the course of human exposure to a wide variety of infections throughout a long history of human evolution, in the region V. Similarly, non-human animals have natural antibodies, though they may be involved in antibody formation specific to individual animals and therefore, their reactivity including cross-reactivity depend on the animal species. It is thought that an human polyclonal antibody, which is obtained by immunizing the non-human animal having a human antibody gene locus with an immunogen containing a plurality of human region V gene segments, is not a replacement with a animal polyclonal antibody, which is commonly obtained simply by immunizing the animal with an immunogen, and so has a nature specific to the human polyclonal antibody different from that of the animal polyclonal antibody. The inventors of the present invention immunized the animals having a human antibody gene locus and the animals having their inherent antibody gene locus with an immunogen on schedule of the same protocol and made a detail examination of the reactivity of obtained individual polyclonal antibodies, the result from which gave us a suggestion of a difference between both groups of animals in immunogenic specificity, especially in cross-reactivity and brought us a successful completion of the present invention.

According to a conventional standpoint, in any animal, an antibody which reacts to an immunogen, can be produced by immunizing with the immunogen. Based on this standpoint, it is difficult to predict whether a polyclonal antibody to a wide variety of antigens other than the immunogen (including) is obtained by immunizing the animal with the immunogen. The inventors of the present invention observed that a difference in the reactivity of the polyclonal antibody obtained by immunizing with the same immunogen existed between a group of animals having a human antibody gene locus and a group of animals having their own inherent antibody gene locus for the first time. This difference may reflect a potential difference between human antibody region V gene segments and non-human antibody region V gene locus segments.

In any animal in which a human antibody having various segments in a human region V can be produced, the natural antibody inherently existing in human bodies is easy to be produced and furthermore, the human polyclonal antibody produced in the animal has the reactivity close to that of the polyclonal antibody produced in human bodies through natural infection. Thus, a human antibody drug with less adverse effects can be provided.

The inventors of the present invention had devoted themselves to the study and finally successfully obtained a human polyclonal antibody having a desired antibody titer against a bacterium and/or virus and a wide spectrum. The antibody can be favorably obtained by administering as an immunogen a bacterium and/or virus to a non-human animal having a human antibody gene locus.

The human polyclonal antibody of the present invention provides a human antibody drug, which can overcome the problems involved with IVIG and MoAb and is applicable to the treatment and prevention of infections.

The human polyclonal antibody of the present invention has an antibody titer against a specific bacterium and/or a virus higher than those of a immunoglobulin drug and/or human pool plasma collected from several thousands of human individuals, from which components are used as a material for the immunoglobulin drug, and an antibody spectrum equal to or wider than the special immunoglobulin derived from human plasma with no strain-specific reactivity sometimes seen in the monoclonal antibody. This means that targetting a certain kind of bacteria and viruses, as an example of the human polyclonal antibodies, human polyclonal antibody compositions has characters that the activities of the individual compositions against the bacterium and/or virus may reflect the relative titers equal to or higher than those of pooled human plasma as based on index of 1) ELISA antibody titer, 2) agglutination titer, or 3) neutralizing antibody titer by the law of the art and they have a wide antibody spectrum so that they have reactivity to not only the serotype of a specific immunogen but also those of other immunogens. The wording of "the relative titer" stated herein means the relative activity of a certain volume of IgG and/or IgM involved in the antibody activity. For example, focusing on Pseudomonas aeruginosa, the human polyclonal antibody compositions of the present invention have relative titers to those of pool human plasma, 1 to 650 times for the ELISA antibody titer, 30 to 550 times for the agglutination titer, and 36 to 150 times for the neutralizing activity, and the compositions indicate reactivity not only to the serotype but also to other serotypes of *Pseudomonas aeruginosa*. For example, in case where a non-human animal is immunized with *Pseudomonas aeruginosa* of serotype A, G, E, B, or I , the composition has the reactivity not only to the immunized serotypes but also to as C, H, and M serotypes. Similarly, focusing on bacteria and cytomegalovirus including *Pneumococcus*, *Escherichia coli*, and *Staphylococcus aureus* and *Japanese encephalitis* virus, the human polyclonal antibody compositions of the present invention have the same characters.

In brief, the present invention can be characterized by:
(1) A human polyclonal antibody composition having the effects of prevention or treatment of infections, including a human polyclonal antibody obtained by administering as immunogen a bacterium or a virus or a component of the a bacterium or a virus to a non-human animal having a human antibody gene locus and the human polyclonal antibody having an antibody titer against the bacterium and/or the virus exceeding that of human pool plasma.;
(2) The human polyclonal antibody composition defined in (1), wherein they have the antibody titer against the bacterium or the virus used as immunogen are higher than that of human pool plasma, at least 1.1 times or more for the ELISA antibody titer, for at least 4.4 times for the agglutination titer, and at least 14 times for the neutralizing antibody titer;
(3) The human polyclonal antibody composition defined in (1) and (2), further containing a polyclonal antibody which recognizes bacteria and/or viruses other than the bacterium and/or the virus used as immunogen;
(4) The human polyclonal antibody composition defined in any of (1) to (3), wherein they are obtained by administering as immunogen at least two kinds of bacteria and/or viruses or components of these bacteria and/or viruses and have reactivity to at least the two kinds of bacteria and/or viruses;
(5) The human polyclonal antibody composition defined in any of (1) to (4), wherein the immunogen to be used is selected out of gram-positive bacteria or gram-negative bacteria;
(6) The human polyclonal antibody composition defined in any of (1) to (5), wherein the immunogen to be used is selected out of a group of *Pseudomonas aeruginosa*, *Pneumococcus*, and *Staphylococcus aureus*;
(7) The human polyclonal antibody composition defined in any of (1) to (6), wherein the immunogen to be used is drug resistance bacteria (a drug resistance bacterium);
(8) The human polyclonal antibody composition defined in (7), wherein, the drug resistance bacterium is MRSA;
(9) The human polyclonal antibody composition defined in any of (1) to (4), wherein they are obtained using immunogen selected out of DNA or RNA viruses;
(10) The human polyclonal antibody composition defined in (9), wherein the immunogen to be used is selected out of cytomegalovirus and Japanese encephalitis virus;
(11) The human polyclonal antibody composition defined in any of (1) to (10), wherein a non-human animal having a human antibody gene locus is the non-human animal having a plurality of human region V gene segments;
(12) The human polyclonal antibody composition defined in (11), wherein a plurality of human region V gene segments include eight or more segments in both of regions VH and VL in total;
(13) A human polyclonal antibody, which contains a human polyclonal antibody having reactivity to a plurality of bacteria and/or viruses including bacteria and/or viruses other than a bacterium or a virus used as immunogen for preventing or treating infections;
(14) The human polyclonal antibody defined in (13), wherein the human polyclonal antibody is obtained by administering as immunogen two kinds of bacteria and/or viruses or components of these bacteria and/or viruses;
(15) A human polyclonal antibody, which is obtained by administering as immunogen bacteria of at least two kinds of serotypes or components of the bacteria of said serotypes to a non-human animal having a human antibody gene locus, the human polyclonal antibody recognizing not only the bacteria of serotype used as immunogen but also the bacteria of serotypes other than the serotype of the bacterium used as immunogen;
(16) The human polyclonal antibody defined in (15), wherein a bacterium is *Pseudomonas aeruginosa*;
(17) The human polyclonal antibody defined in (16), wherein the human polyclonal antibody is obtained by administering *Pseudomonas aeruginosa* of serotypes 122, IT-1, IT-2, IT-3, and IT-4 to a non-human animal having a human antibody gene locus and has reactivity to *Pseudomonas aeruginosa* of serotypes 122, IT-1, IT-2, IT-3, IT-4, IT-5, IT-6, IT-7, and IF03080;
(18) The human polyclonal antibody defined in any of (13) to (17), wherein the non-human animal having a human antibody gene locus is the non-human animal having a plurality of human region V gene segments;
(19) The human polyclonal antibody defined in (18), wherein a plurality of human region V gene segments include eight or more gene segments in both of regions VH and VL in total;
(20) The human polyclonal antibody composition, which contains the human polyclonal antibody defined in any of (13) to (19), for preventing or treating infections;
(21) The human polyclonal antibody compositions defined in any of (1) to (13) and (20), wherein they are used as a substitute for an immunoglobulin drug;
(22) A method for manufacturing the human polyclonal antibody defined in any of (14) to (19), comprising a step for immunizing the non-human animal having a human antibody gene locus with a bacterium and/or virus or a component of the bacterium and/or the virus;
(23) The method for manufacturing the polyclonal antibody compositions defined in any of (1) to (13) and (20), comprising a step for immunizing the non-human animal having a human antibody gene locus with bacteria and/or viruses or a component of the bacteria and/or the viruses;
(24) The human polyclonal antibody composition for preventing or treating infections by a bacterium and/or a virus, the human polyclonal antibody having higher antibody titer than that of human pool plasma, at least 1.1 times or more for the ELISA antibody titer, at least 4.4 times or more for the agglutination titer, and at least 14 times or more for the neutralizing antibody titer;
(25) The human polyclonal antibody composition defined in (24), wherein the bacterium is *Pseudomonas aeruginosa*;
(26) The human polyclonal antibody composition defined in (25), wherein *Pseudomonas aeruginosa* includes *Pseudomonas aeruginosa* of two kinds or more of serotypes;
(27) The polyclonal antibody composition defined in (26), wherein *Pseudomonas aeruginosa* includes *Pseudomonas aeruginosa* of serotypes 122, IT-1, IT-2, IT-3, IT-4, IT-5, IT-6, IT-7, and IFO3080; and
(28) The human polyclonal antibody composition defined in any of (24) to (27), wherein the compositions are used as a substitute for an immunoglobulin drug.

Now, The present invention is described in detail.

The human polyclonal antibody of the present invention preferably can be prepared from plasma and/or serum obtained from the non-human animal having a human antibody gene locus, to which an immunogen, from which a desired antibody activity is acquired, is immunized.

The non-human animal having a human antibody gene locus can be generated by the techniques described below.

Mouse having a human antibody gene locus can be generated by performing the steps; first, an antibody knock-out (KO) mouse with its inherent antibody H chain and κ chain gene locus made dysfunctional is generated using the embryonic stem cell by gene targeting and then, human antibody H chain and L chain gene loci are introduced into a fertilized egg or into a mouse ES cell using the artificial chromosome of an yeast(N.Lonberg et al, Nature, vol.368, p.856, 1994; L.L.Green et al, Nat. Genet., vol.7, p.13, 1994).

In addition, first by increasing the number and/or the kind of a human region V gene to be introduced into the mouse using the artificial chromosome system of the yeast and finally by introducing 66 VH gene segments and 32 Vκ gene segments, the mouse having a human antibody production system compatible with variety in a human body can be generated (M.J.Mendez et al, Nat.Genet., vol.15, p.146, 1997).

The human antibody producing animal generated in this way is referred to as a trans-genic (TG) animal herein and the method for generating the trans-genic animal is referred to as the TG technique.

In the human antibody gene locus, giant clusters are formed; more than about 1.5 Mb on the chromosome #14 in the H chain and more than about 2 Mb on the chromosome #2 for the κ chain and more than about 1 Mb on the chromosome #22 for the λ chain in the L chain; and it is difficult to introduce a complete-length of antibody gene locus even using said artificial chromosome. Alternatively, the mouse having the almost complete human antibody gene locus (human chromosomes #14 and #2) can be generated from the mouse ES cell, into which the human chromosomes are introduced by micro cell fusion (K. Tomizuka et al, Proc. Natl. Acad. Sci. USA, vol.97, p.722, 2000). Thus, the mouse, into which the chromosomes have been introduced, is trans-chromosomic (TC) mouse. In this specification, this method is referred to as the TC technique and the animal generated by this method is referred t as the TC animal.

From an aspect of an antibody spectrum, the non-human animal having a human antibody gene locus, which is used in this present invention, desirably contains a plurality of human region V gene segments. Furthermore, considering that such cases have been reported that useful human antibodies could be prepared (N.Lonberg et al, Nature, vol.368, p.856, 1994; L.L.Green et al, Nat. Genet., vol.7, p.13, 1994) , that a new sequence could be inserted into even the region V having a limited number of region V gene segments during re-sequencing (S. Tonegawa,Nature,vol.302,p.575,1983), and that after re-sequencing, a somatic mutation changed immunogenic specificity, it is desired that the non-human animal having a human antibody gene locus, which is used in this present invention, contains eight or more regions human region V gene segments in both of the VH and VL regions in total.

For example, the regions V of the mice generated by the TG technique described above have four and five gene segments in the region VH and four and three gene segments in the region Vκ.

It may be possible to use both of TG and TC animals as the non-human animals having a plurality of human region V gene segments, though at present, it is desirable to use the TC animal, into which more human region V gene segments can be introduced. The present invention, however, can be achieved by any method not limited to the methods described above for introducing the human antibody gene locus into the non-human animal. In addition, the cells, into which human antibody gene segments are introduced, are not limited to ES cells or somatic cells and in the present invention, any of techniques, which can generate the non-human animal having the gene segments constituting a plurality of human regions V, may be used.

In the present invention, the non-human animal having the human antibody gene locus is not limited and any of non-human animals, into which the human antibody gene locus can be introduced, may be used. For example, such a non-human animal includes a mouse, rat, swine, goat, ovine, and bovine.

Moreover, the polyclonal antibody of the present invention can be obtained by immunizing the non-human animal having the human antibody gene locus with a bacterium and/or a virus or a component of the bacterium and/or the virus. At this time, it is desirable that a plurality of immunogens of at least two kinds or more are mixed to immunize the non-human animal.

By mixed immunization using a plurality of immunogens, the human polyclonal antibody produced in the human antibody producing animal having cross-reactivity to immunogenes other than the immunogen administered, a wider spectrum, and higher effects of treatment can be obtained. It has not been known that the polyclonal antibody produced in the human antibody producing animal has such cross-reactivity and is disclosed in the specification of the present invention for the first time.

The immunization of a single immunogen does not allow the derivation of a human polyclonal antibody having such cross-reactivity with higher effects of treatment. No cases have been known that TC and/or TG animal are immunized with a plurarity of immunogens to obtain a human polyclonal antibody with higher cross-reactivity. The objectives of the cases reported so far were to obtain a human MoAb with immunogenic specificity and high affinity to the immunogen. For the objection, the immunization with a single immunogen was selected. Therefore, the human polyclonal antibody obtained by the conventional method having similar characters to that of a human MoAb cannot be a drug for preventing or treating common infections, most of which are mixed infections by bacteria of various serotypes or bacteria and/or viruses of different kinds as explained in the case of a human MoAb.

As the method for mixed immunization described above, either the method in which all the immunogens are mixed together to be used for immunization at the same time or the method in which individual immunogens are used sequentially for immunization, can be used. In either method, the human polyclonal antibody with intended characters can be obtained. Alternatively, a plurality of serotypes of pathogen of the same kind, different pathogens of a plurality of kinds, or the mixture of both of these pathogens can be used for immunization.

Besides, the kind of the immunogen is not limited and depending on the application, various pathogens can be used as immunogens. For example, the pathogens, which can be used as immunogens, include gram-positive bacteria including *Pseudomonas aeruginosa, Pneumococcus*, and *Escherichia coli* or DNA viruses and RMA viruses including *Cytomegalovirus* and Japanese encephalitis virus. Similarly, drug resistance bacteria including MRSA can be used as an immunogen. By selecting a plurality of kinds out of these bacteria and/or viruses and immunizing the non-human animal having the human antibody gene locus with them, the human polyclonal antibody of the present invention can be obtained.

Alternatively, bacteria of the same kind but of different serotypes may be mixed to be used for the immunization of the non-human animal. *Pseudomonas aeruginosa* and *Pneumococcus* are known as the bacteria having serotypes.

For example, the human polyclonal antibody can be obtained by immunizing the non-human animal with a plurality of *Pseudomonas aeruginosa* strains of different serotypes.

With no special limitation, the quantities of immunogens to be used for immunization can be determined as necessary depending on the kind of a non-human animal having a human antibody gene locus. In addition, the interval of immunization can be determined as necessary. Concerning the method for immunizing with immunogens, any one may be used provided that it can be commonly used in immunizing the non-human animal. For example, the immunogens can be used to immunize the non-human animal through any of the routes, subdermal, intraperitoneal, intravenous, intramuscular, and intracutaneous. It is desirable that the immunogens are used to immunize the non-human animal with the addition of a commercially available Freund's complete adjuvant, Freund's incomplete adjuvant, or an appropriate adjuvant including BCG, aluminum hydroxide gel, and *Pertussis* vaccine.

In this way, a human polyclonal antibody derived from human pool plasma, namely a human polyclonal antibody having a high titer equal to or higher than that of a human globulin drug and a wide spectrum, can be obtained using a non-human animal having a human antibody gene locus.

Human polyclonal antibody compositions produced in the non-human animal can be prepared into a drug, which can be put on the market and distributed as an immunoglobulin drug, by means of the same purification process as that of normal IVIG. Specifically, the drug of the present invention can be prepared by preparing an IgG fraction from the blood of the non-human animal by the method, for example Cohn's ethanol fractionation (Immunoglobulin Therapy, edited by Katsutoshi KOMURO, Kindai Shuppan, P.222, 1992) and various types of chromatographic techniques (including ion-exchange chromatography, hydrophobic chromatography, gel filtration chromatography, affinity chromatography, and reverse phase chromatography) and then performing as necessary pepsination, plasmination, sulfonation, alkylation, pH4 processing, polyethylene glycol processing, and freeze-drying processing.

The human polyclonal antibody obtained from a non-human animal can be prepared into a drug by diluting with for example physiological saline or a buffer solution. It is desirable that the pH of the drug lies within a acidulous to neutral range, which is close to the pH of body fluid. Its lower limit lies within a pH 5.0 to 6.4, and its upper limit lies within a pH 6.4 to 7.4. Furthermore, the drug of the present invention can be provided in the form capable of being stored for a long period, such as freeze-dried, wherein it can be used as a immunoglobulin drug by dissolving with for example water, physiological saline, or a buffer solution for dilution to the desired concentration.

The drug of the present invention may contain additives (for example, a carrier, vehicle, and diluent), stabilizing agent, an other pharmaceutically required component provided that they are pharmacologically accepted. The stabilizing agent includes monosaccharide for example glucose, disaccharide for example saccharose and maltose, sugar alcohol for example mannitol and sorbitol, neutral salt for example sodium chloride, amino acid for example glycin, nonionic surfactant for example polyethylene glycol, polyoxyethylene-polyoxypropylene copolymer (PLURONIC), and polyoxyethylene sorbitan fatty acid ester (Tween), and human albumin and any of them is preferably added at any level between about 1 to 10 w/v%.

When the drug of the present invention is intravenously injected to, for example a patient with a infection for treatment, generally 1000 to 10000 mg/injection (several tens to hundreds mg/weight of 1 kg) is administered to an adult patient with a increment or decrement depending on the patient's condition and age. An effective volume of drug of the present invention can be administered by means of intravenous or intramuscular injection.

The human polyclonal antibody of the present invention is characterized in that it has an ELISA antibody titer, an agglutination titer, and a neutralizing antibody titer against a wide range of bacteria, bacterial toxin, and viruses equal to or higher than those of a human immunoglobulin drug for example immunoglobulin for intravenous injection or human pool plasma used as the material for the drug. Various types of human globulin drugs such as immunoglobulin for intravenous injection are commercially available including dried, sulfonated human immunoglobulin (proprietary name: Venilon, prepared by Chemo-Sero-Therapeutic Research Institute) and human immunoglobulin (proprietary name: Chemo-Sero-Therapeutic Research Institute-gammaglobulin injection solution 15%, prepared by Chemo-Sero-Therapeutic Research Institute). Human pool plasma is the mixture of human plasma collected from several thousands of human individuals and used as the material for a human immunoglobulin drug. Human pool plasma may be generally referred to as plasma and in this specification, the word of plasma stated means human pool plasma described above in some cases.

A wide range of bacteria and viruses include gram-positive germs and gram-negative germs including *Pseudomonas aeruginosa*, *Pneumococcus,* and *Escherichia coli* and DNA viruses and RNA viruses including *Cytomegalovirus* and *Japanese encephalitis* virus.

The ELISA antibody titer, agglutination titer, and neutralizing antibody titer of the human polyclonal antibody of the present invention are measured using a plural kind of bacteria and/or viruses used as immunogens, and a plural kind of bacteria and/or viruses other than the immunogens(gram-positive bacteria or gram-negative bacteria) as antigens. Based on the ELISA antibody titer, agglutination titer, and neutralizing antibody titer of the polyclonal antibody of the present invention, its efficacy as an immunoglobulin drug can be determined. The bacteria and viruses used as antigens are not limited but in addition to bacteria and viruses used as immunogens, using for example, *Pseudomonas aeruginosa*, *Cytomegalovirus*, *Japanese encephalitis* virus, *Pneumococcus*, *Escherichia coli*, and *Staphylococcus aureus*, the antibody titer, agglutination tier, and neutralizing antibody titer of the polyclonal antibody of the present invention can be measured. The ELISA antibody titer can be measured by conventional ELISA. For example, first, a bacterium or a virus as it is or the component (any of roughly purified products and purified products can be used) of the bacterium or the virus are solidified on a commercially available 96-well micro-titer plate. At this time, the volume of the bacterium or the virus to be solidified is not limited but if it is solidified at for example, the level of several to several tens ng/well equivalent to the protein mass, the ELISA antibody titer can be better measured. Then, the human polyclonal antibody of the present invention or human pool plasma, which is prepared into a human immunoglobulin drug or its component, is added to the well as a sample, wash the well after a certain time period, a secondary antibody with labeled with an appropriate coloring enzyme (antibody against human immunoglobulin) is added, and after a certain time period of reaction, the well is washed out. After then, the substrate of the enzyme is added for coloring reaction and the level of coloring is read out using a micro plate reader and others to measure the reactivity of the solidified antigen and the sample. At this time, by gradually diluting the sample for preparing the dilution sries, the ELISA antibody titer can be represented by a multiple factor of dilution at which the sample reacts to the solidified antigen or coloring higher than a certain level is observed. Using the value obtained by the ELISA antibody titer subtracted by the immunoglobulin content (IG content) or the immunoglobulin concentration (IG concentration) of the antibody in the well, the ELISA antibody titer can be compared among different samples. In addition, the ELISA antibody titer cab be represented by the IG content or the IG concentration in the well where a certain level of coloring. For example, assuming that OD=0.05 to 0.5, the ELISA antibody titer can be represented by the IG concentration(µg/ml). The IG content can be measured by techniques such as IgG and IgM measurement ELISA. In measuring the ELISA antibody titer, a secondary antibody, which reacts only to human immunoglobulin IgG or to both of human immunoglobulin IgG and IgM, may be used. The ELISA antibody titer of the polyclonal antibody of the present invention measured this way is compared with that of the antibody titer of immunoglobulin or human pool plasma. Similarly, the agglutination titer can be measured by means of the agglutinationsuch as a bacterium agglutination, a hemagglutination, and a agglutination using a carrier for example latex. For example, the body of the bacterium is mixed with any of the samples such as the polyclonal antibody of the present invention, a immunoglobulin drug, and human pool plasma, added to the 96-well U-shaped bottom plate, left as it is for a certain time period, and then agglutination tier can be measured by observing the agglutination level of the body of the bacterium. In addition, The agglutination may be performed by absorbing the component of the bacterium or the virus to the carriers of red cells or latex beads and mixing with the sample. At this time, by gradually diluting the sample for obtaining the dilution line, the coagulation value can be represented by the multiple factor of dilution at which agglurination is observed. The IG content (IG concentration) in the sample is measured by IgG and IgM ELISA and the agglutination titer/unit IG content (IG concentration) are compared. In this case, it is preferable that the IG volume is measured by both of IgG ad IgM ELISA. The agglutination titer of the polyclonal antibody of the present invention measured this way is compared with that of immunoglobulin or human pool plasma.

The neutralizing antibody titer can be measured by finding the fatality rate or the incidence rate of the animals such as mice, to which any of samples such as the polyclonal antibody of the present invention or a immunoglobulin drug (or pool plasma used as a material component) administered with a bacterium or a virus. The sequence of the administration of the sample and the bacterium or the virus is not limited and they may be administered together at a time. They can be administered to a plurality of individual animals and based on the number of the survival animals or the animals in which no infection has developed, the neutralizing antibody titer can be measured. At this time, a group of animals, to which only a bacterium or a virus is administered with no polyclonal antibody used, is set as a control group and the results are compared between the treated group and the non-treated group, enabling the determination of the effects of the polyclonal antibody. For example, the polyclonal antibody of the present invention or human pool plasma is administered to a given number of mice followed by *Pseudomonas aeruginosa* and after several days pass, the number of survival mice is counted. The neutralizing antibody titer of the polyclonal antibody of the present invention can be calculated by comparing the IG content or the IG concentration of the polyclonal antibody of the present invention, in which a neutralizing activity is observed relative to those of the control groups, to which immunoglobulin, a immunoglobulin drug, or human pool plasma, with the IG content or the IG concentration of the control group, in which no neutralizing activity is observed.

Moreover, in the case of viruses, the neutralizing antibody titer can be measured in vitro in the way mentioned below. The virus, the polyclonal antibody of the present invention, and a immunoglobulin a drug or pool plasma are mixed followed by cultured cells such as Vero cells and after a certain time period of cultivation, the number of plaques, which have been formed by infection with the virus is counted. At this time, the dilution lines of the polyclonal antibody, the immunoglobulin drug, and pool plasma have been prepared by means of gradual dilution and then, the dilution factor indicating the half of the plaques formed in the culture plate, to which no polyclonal antibody is added, can be assumed to be the neutralizing antibody titer. The neutralizing antibody titer of the polyclonal antibody of the present invention measured this way is compared with that per IG of immunoglobulin or human pool plasma.

### BRIEF DESCRIPTION OF DRAWINGS

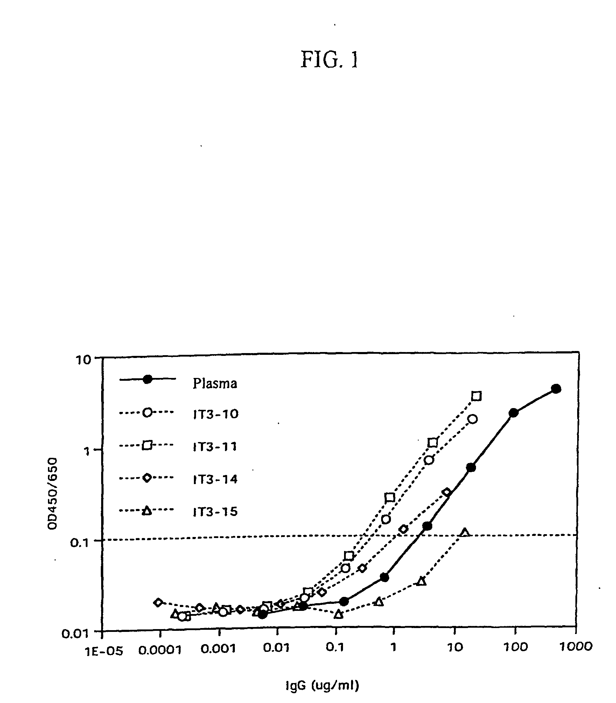

FIG 1 is a diagram of the result from the experiment that the anti-*Pseudomonas aeruginosa* human antibody contained in serum obtained by immunizing TC mouse with a *Pseudomonas aeruginosa* IT-3 strain was detected by ELISA.

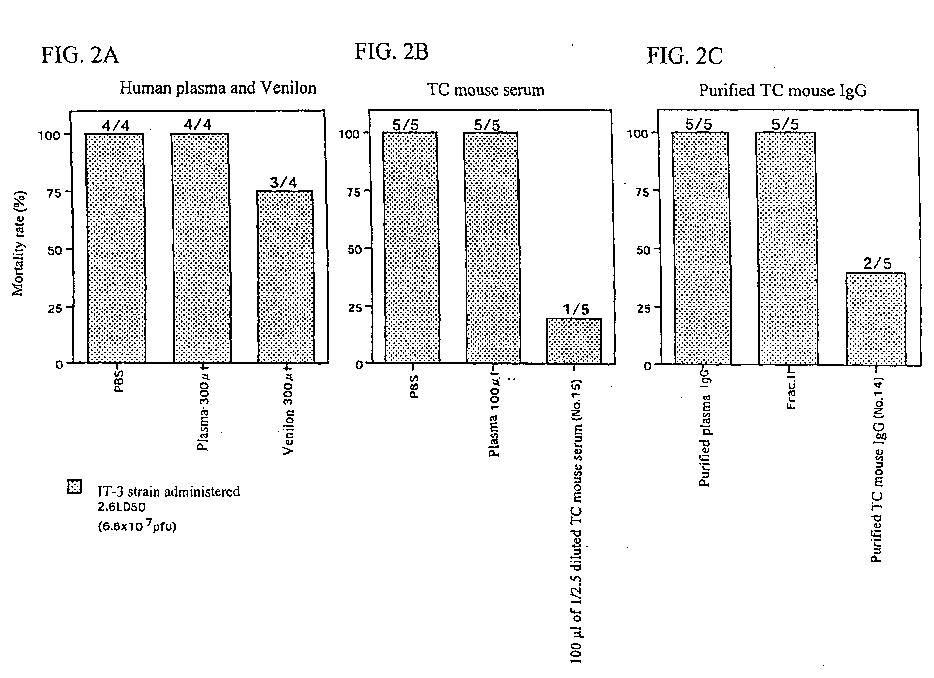

FIG 2 is a diagram of the result from the experiment that the in vivo neutralizing activity of the anti-*Pseudomonas aeruginosa* human antibody contained in serum obtained by immunizing TC mouse with a *Pseudomonas aeruginosa* IT-3 strain was detected. FIG 2A shows the case of human plasma and Venilon, FIG. 2B shows the case of the TC mouse serum, and FIG 2C shows the case of purified TC mouse IgG.

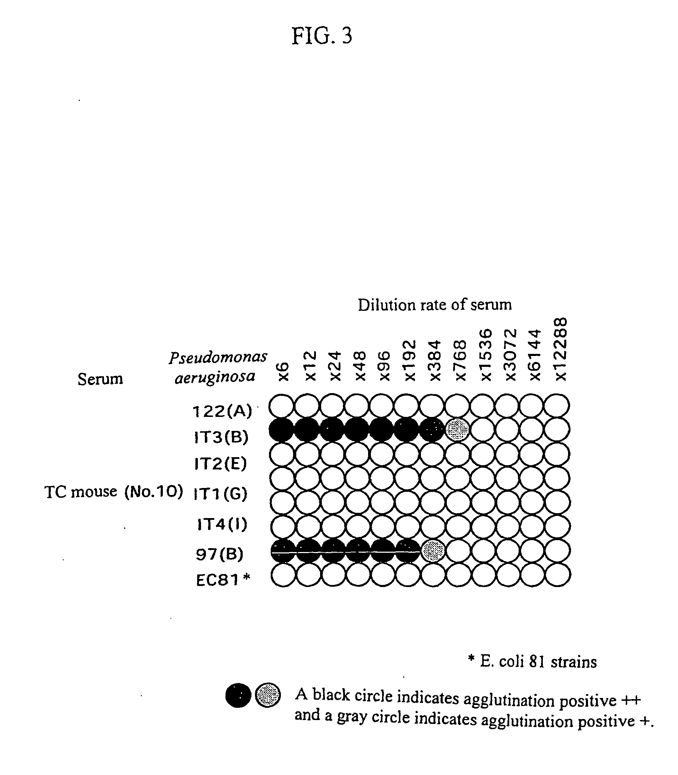

FIG.3 is a diagram of the result from the experiment that the agglutination activity of the anti-*Pseudomonas aeruginosa* human antibody contained in serum obtained by immunizing TC mouse with a *Pseudomonas aeruginosa* IT-3 strain was detected. In the figure, a black circle indicates an agglutination positive ++ and a gray circle indicates an agglutination positive +, respectively.

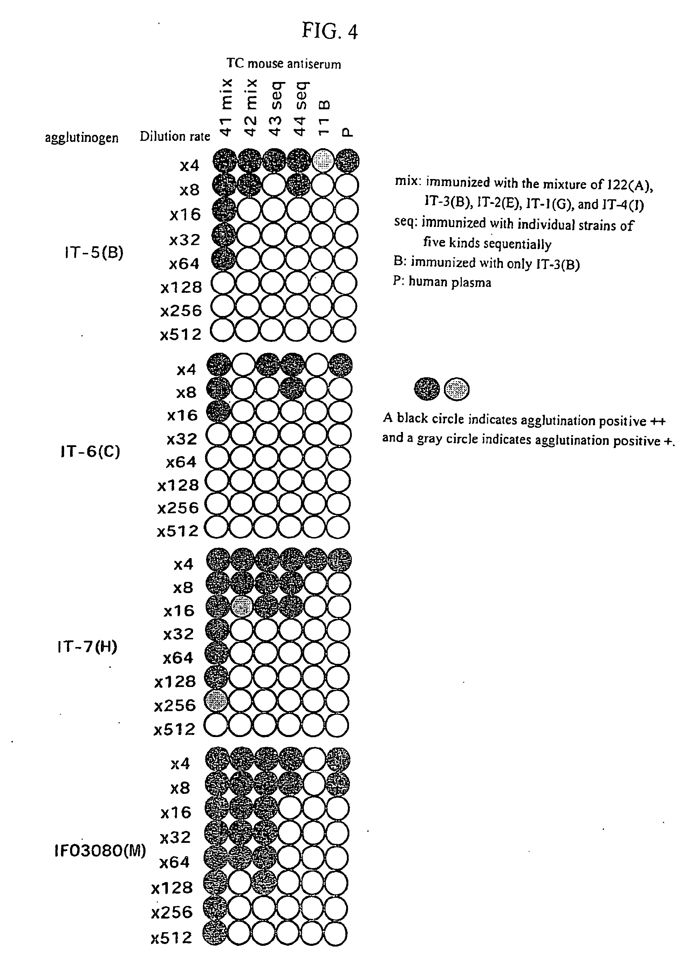

FIG 4. is a diagram of the result from the experiment that the agglutination activity of the anti-*Pseudomonas aeruginosa* human antibody contained in serum obtained by immunizing TC mouse with different *Pseudomonas aeruginosa* of a plural kind was detected. The agglutinogen IT-5(B), IT-6(C),IT-7(H), and IFO3080(M) were used starting from the upper side. In the figure, "mix" indicates that 122(A), IT-3(B), IT-2(E), IT-1(G), and IT-4(I) were mixed to be used for immunization of the animals. "Seq" indicates that individual strains of five different kinds were separately used for immunization of the animals. "B" indicates that only IT-3(B) was used for immunization of the animals. "P" is human plasma. In the figure, a black circle indicates an agglutination positive ++ and a gray circle indicates an agglutination positive +, respectively.

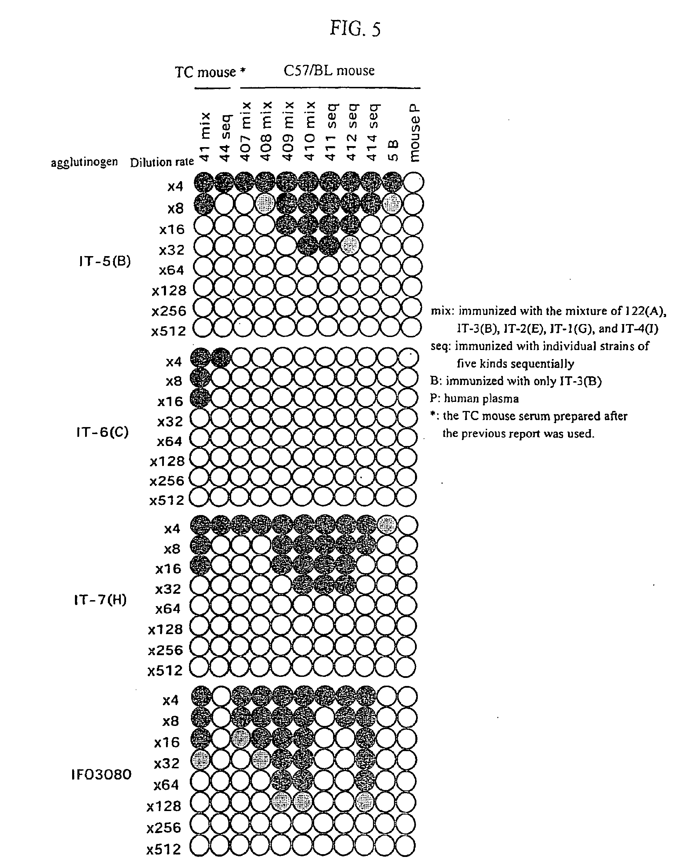

FIG.5 is a diagram of the result from the comparison in agglutination activity between the anti-*Pseudomonas aeruginosa* human antibody contained in TC mouse serum and C57/BL mouse serum obtained by immunizing the mice with different *Pseudomonas aeruginosa* of a plural kind, and the anti-*Pseudomonas aeruginosa* mouse antibody. The agglutinogens IT-5(B), IT-6(C), IT-7(H), and IFO3080 were used starting from the upper side in the figure. In the figure, "mix" indicates that 122(A), IT-3(B), IT-2(E), IT-1(G), and IT-4 (I) were mixed to be used for immunization of the animals. "Seq" indicates that the individual strains of five different kinds were separately used for immunization of the animals. "B" indicates that only IT-3(B) was used for immunization of the animals. "P" is human plasma. In the figure, a black circle indicates an agglutination positive ++ and a gray circle indicates an agglutination positive +, respectively.

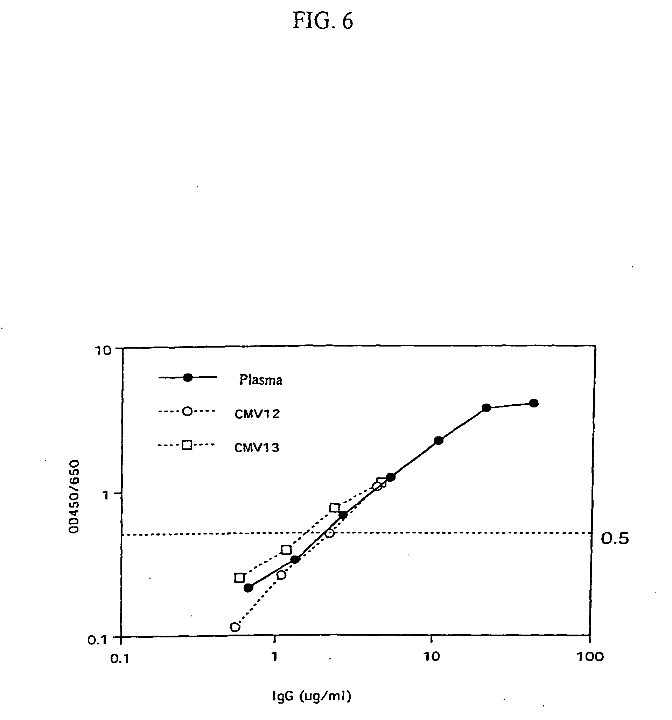

FIG. 6 is a diagram of the result from the experiment that anti- *Cytomegalovirus* human antibody contained in serum obtained by immunizing TC mouse with a *Cytomegalovirus* AD 169 strain, was detected by ELISA.

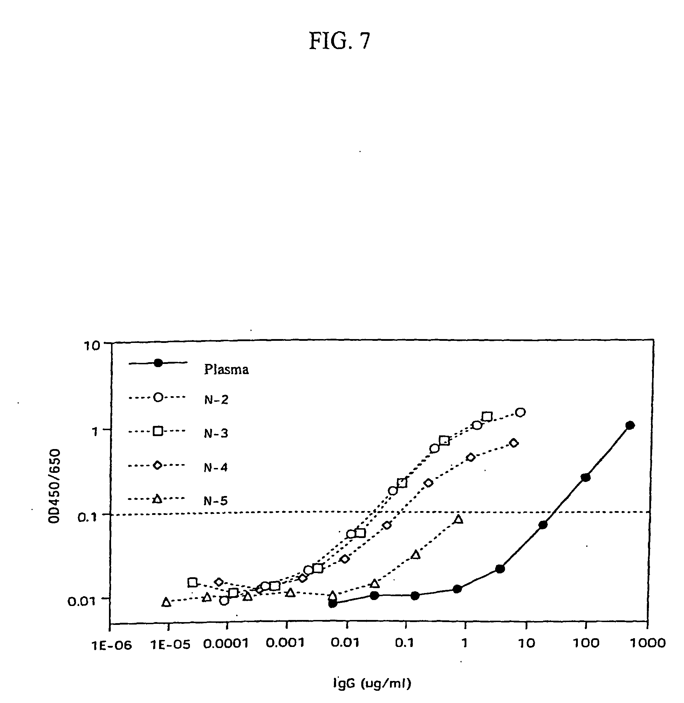

FIG.7 is a diagram of the result from the experiment that anti- *Japanese encephalitis* virus human antibody contained in serum obtained by immunizing TC mouse with a *Japanese encephalitis* inactivated, purified immunogen was detected by ELISA.

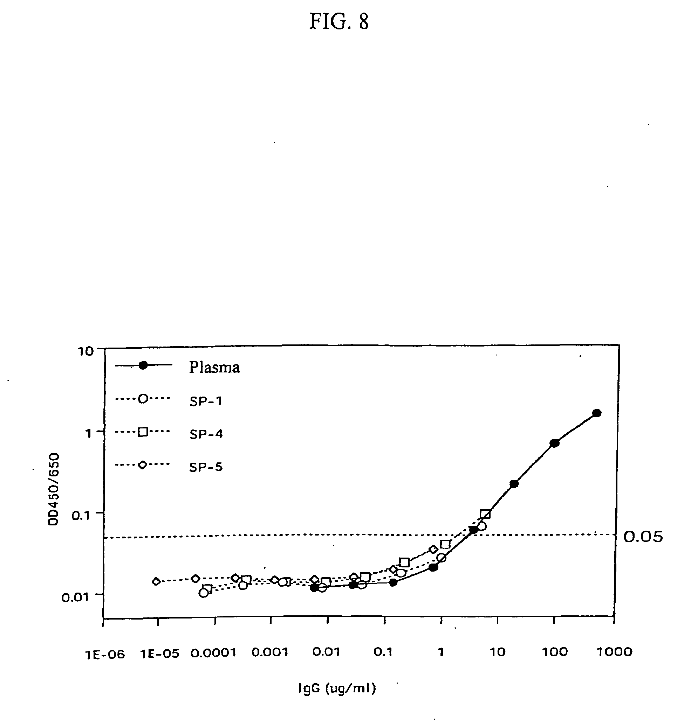

FIG.8 is a diagram of the result from the experiment that the anti- *Pneumococcus* human antibody contained in serum obtained by immunizing TC mouse with a *Pneumococcus* DIII 15A strain was detected by ELISA.

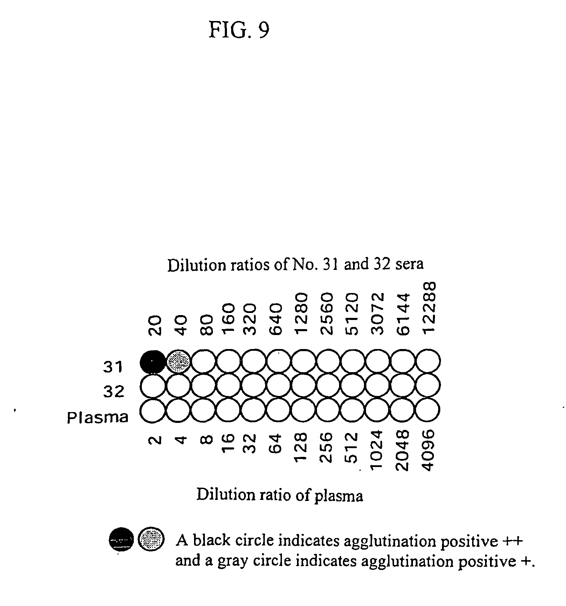

FIG.9 is a diagram of the result from the experiment that the agglutination activity of the anti- *Escherichia coli* human antibody contained in serum obtained by immunizing TC mouse with a *Escherichia coli* 81 strain was detected. In the figure, a black circle indicates an agglutination positive ++ and a gray circle indicates an agglutination positive +, respectively.

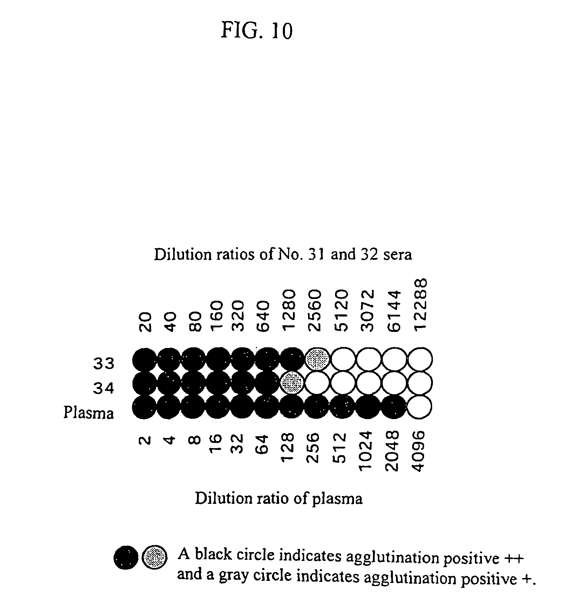

FIG.10 is a diagram of the result from the experiment that the agglutination activity of the anti- MRSA human antibody contained in serum obtained by immunizing TC mouse with MRSA was detected.

In the figure, a black circle indicates an agglutination positive ++ and a gray circle indicates an agglutination positive +, respectively.

### BEST MODE FOR CARRYING OUT THE INVENTION

Now, the present invention is described in detail by referring the embodiments of the present invention. Note that the technical scope of the present invention is not limited to but includes the examples.

### Example 1 Generating a human antibody producing TC mouse

The mice described in the report by Tomitsuka et al., (Proc.Natl.Acad.Sci.USA., vol.97, p.722,2000), in which mouse endogeneous antibody heavy chain and a κ light gene were destructed and a human chromosome #14 fragment containing the entire region of a human antibody heavy chain gene locus and a human chromosome #2 containing the entire region of a human antibody light chain κ gene locus were retained, were used in the experiment described below. The mice do not express antibody heavy chain and κ light chain and the primary compositions of the antibody detected in the serum were complete human antibodies (IgG, IgM, and others) consisting of a human heavy chain and a human κ light chain.

### Example 2 Producing an anti-Pseudomonas aeruginosa antibody

### 1) Immunization with Pseudomonas aeruginosa

A *Pseudomonas aeruginosa* IT-3 strain (S.Sawada et al, J. Gen. Microbiol., vol.133, p.3581, 1987) was cultured on the Trypticase Soy Agar(BBL) at 37°C over night, collected and suspended in a PBS. Then, the susupension was added with hormalin up to 1%, and inactivated for a time period of 24 hours or more to be used in the experiment.

Initially, the strain was intrapenitreally administered to the TC mice described in the embodiment 1 with a Freund's complete adjuvant and then, administered at an interval of two weeks with an incomplete adjuvant four times in total. The group immunized with 100 µG/mouse of IT-3 strain (No.10.11) and the group immunized with a mixture of IT-3 strain (100µg/mouse) and Cytomegalovirus AD 169 strain (see the example 3: 1.2×10⁶PFU/mouse) (No.14,15) were set. After immunization of the immunogen, blood was partially collected from the vein of the mouse tail or blood was completely collected from the mouse heart, the collected blood was left as it was at room temperature for a time period of two hours, centrifuged (9,000G, 15 minutes), and then supernatant produced was collected as serum (similarly, immune sera described in the succeeding examples were prepared).

### 2) ELISA antibody titer

1% of hormalin inactivated IT-3 strain was solidified on an ELISA plate(MaxiSorp type, NUNC), HRP-labeled anti-human IgG antibody (SIGMA) was used as a secondary antibody, and the antibody titer against the IT-3 strain of IT-3 strain administered TC mouse serum (No.10,11,14,15) was measured by the method of Sawada et al. (S.Sawada et al, J. Gen. Microbiol., vol.133, p.3581, 1987) (similarly, ELISA described in the succeeding examples were performed with varying the solidified immunogen. The result is shown in FIG.1. In addition, IgG measurement ELISA(K. Tomizuka et al, Nat. Genet., vol.16, p.133, 1997) was performed to measure the IgG content in each TC mouse serum, and the ELISA antibody titers were measured for the individual IgG content as shown in FIG.1 (Similarly, the ELISA antibody titer was calculated in the succeeding examples).

**<Table 1>**

| ELISA antibody titer against *Pseudomonas aeruginosa* | | | |
|---|---|---|---|
| Sample | Immunogen | IgG content (µg/ml) for OD = 0.1 | Ratio to plasma |
| Plasma | - | 2.49 | 1.0 |
| No. 10 | IT-3 | 0.40 | 6.3 |
| No. 11 | IT-3 | 0.29 | 8.6 |
| No. 14 | IT-3&CMV | 1.09 | 2.3 |
| No. 15 | IT-3&CMV | 12.14 | 0.2 |

As a result, the ELISA antibody titer of the group of TC mouse immunized with only a IT-3 strain was 6.3 to 8.6 times that of pool plasma (provided by Chemo-Sero-Therapeutic Research Institute)obtained by mixing plasma obtained several thousands of human individuals which is a material for a commercially available immunoglobulin drug. The ELISA antibody titer of the TC mouse immunized with the mixture with CMV was 0.2 to 2.3 times that of the plasma. This suggested that the serum with a ELISA antibody titer higher than pool human plasma capable of being used as a material for immunoglobulin drug could be obtained.

### 3) Agglutination titer

Using the *Pseudomonas aeruginosa* IT-3 strain, the agglutination titer of the serum of the TC mice of the IT-3 strain immunized group (No.10,11,14,15) was measured. The 1% of hormalin inactivated *Pseudomonas aeruginosa* IT-3 strain was diluted by a 1% BSA, 50mM Tris, 0.15M NaCl solution(pH8.0) until it became 2mg/ml, mixed with the same amount (10 µl each) of serum from TC mouse immunized with IT-3 strain, which was gradually diluted by the same solution, on the 96-well U-shaped plate, left at room temperature over night, and then the agglutination titer of bacterial bodies was determined. The result is shown in Table 2. In the plasma group, agglutination was observed in the sample diluted up to two times while in the serum from immunized TC mouse group, it was observed in the sample diluted up to 80 to 320 times.

**<Table 2>**

| Agglutination titer of serum from TC mouse immunized with IT-3 | | | | | | |
|---|---|---|---|---|---|---|
| Sample | No., sexuality, Krin's No. | Immunization | Agglutination titer | IG centent IgG+IgM (mg/ml) | Agglutination titer/IG | Ratio to plasma |
| Plasma | | | 2 | 10.40 | 0.19 | 1 |
| Venilon | | | ND | 56.30 | - | - |
| Blood serum | 10, female, HKD160 | pre-immunization | ND | 0.80 | - | - |
| | | IT-3 | 320 | 3.05 | 104.92 | 552 |
| | 11, female, HKD136 | pre-immunization | ND | 0.94 | - | - |
| | | IT-3 | 160 | 2.45 | 65.31 | 344 |
| | 14, male, HKD138 | pre-immunization | ND | 1.32 | - | - |
| | | IT-3&CMV* | 80 | 2.20 | 36.36 | 191 |
| | 15, male, HKD134 | pre-immunization | ND | 0.92 | - | - |
| | | IT-3&CMV | 80 | 2.01 | 39.80 | 209 |
| Purified IgG | Plasma | | ND | 1.56** | - | - |
| | 10, female, HKD160 IT-3 | | 8 | 0.92** | 8.70 | 46 |
| | 14, male, HKD138 IT-3&CMV | | 8 | 1.26** | 6.35 | 33 |
| ND: no coagulation value | | | | | | |
| -: cannot be calculated | | | | | | |
| *: three times of immunization | | | | | | |
| **: Since purified IgG was used, only the IgG content is indicated. | | | | | | |

Using IgG and IgM measurement ELISA described in a publication (K. Tomizuka et al,Nat. Genet., vol.16, p.133, 1997), IG content in the serum of individual immunized TC mouse was measured. The IgG content in a fraction of plasma, Venilon (provided by Chemo-Sero-Therapeutic Research Institute), and fraction II (Frac.II: IgG fractions prepared from pool plasma by Corn's Fragmentation, provided by Chemo-Sero-Therapeutic Research Institute) used as a control in the ELISA system was 8.9,56.3,and 21.8mg/ml, respectively. IgM content in plasma was 1.50 mg/ml. The agglutination titer per IG(IgG+IgM) content was 0.19 in the plasma and 36.36 to 104.92 in the serum from the immunized TC mouse. The ratio of the serum from immunized TC mouse to the plasma was 191 - 552. In addition, to measure the agglutination titer, in which only IgG was involved, IgG was purified using NAb Protein Spin Chromatography kit(PIERCE) obtained from serum of mouse immunized with IT-3 strain (No.10,14). Then, the agglutination was performed using the IgG. As shown in FIG.2, eight-fold agglutination titer was observed (With respect to the agglutination titer per IG content, the ratio of the IgG to plasma was 33-46 ).

### 4) Neutralizing antibody titer

A challenge test was performed on the neutralizing ability of immunized serum against the IT-3 strain using ICR mouse (Japan SLC). The individual antibody sample was intraperitoneally administered to 5-week old ICR mouse. After 30 minutes, viable cells 2.6LD50(6.6×10⁷cfu) of IT-3 strain suspended in 100µl of PBS were peritoneally admininistered, and the neutralizing activity was determined based on whether they lived or died after four days. The result is shown in FIG.2. Most of mice, to which PBS, plasma, or Venilon which were negative control was administered, died. In this test, no neutralizing activity was observed. While in the group of mice, to which 100µl of serum from TC mouse immunized with IT-3 strain diluted to 1/2.5 times(No.15, IT-3 strain, coagulation value 80) was administered, four of five mice survived. In this test, the neutralizing activity was observed. Concerning the groups of mouse, to which 20µg/100µl of IgG purified from the serum from TC mouse immunized with the IT-3 strain(No.14), 20µg/100µl of purified plasma, or 2.18mg/100µl of Frac.II was administered, all the five mice of the purified plasma group and the Frac.II group died on day 4 after administration but in the group of mouse to which IT-3 strain was immunized, three of five mice survived, meaning that the neutralizing activity was observed. Table 3 shows the ratio of the neutralizing antibody titer to the plasma.

**<Table 3>**

| Comparison of neutralizing antibody titer of the TC mouse antibody against *Pseudomonas aeruginosa* IT-3 | | | | | | | | | |
|---|---|---|---|---|---|---|---|---|---|
| Sample | IG volume*(mg/ml) | Dilution ratio | Administration vol.(µl) | Quantity of administered IG content (mg) | Mortality ratio | Ratio to plasma | | Ratio to Venilon (300µl) | Ratio to Frac.II |
| | | | | | | (100µl) | (300µl) | | |
| PBS | 0.00 | 1 | 300 | 0.00 | 100 | - | - | - | - |
| Plasma | 10.40 | 1 | 300 | 3.12 | 100 | - | 1.0 | - | - |
| Venilon | 56.30 | 1 | 300 | 16.89 | 75 | - | - | 1.0 | - |
| PBS | 0.00 | 1 | 100 | 0.00 | 100 | - | - | - | - |
| Plasma | 10.40 | 1 | 100 | 1.04 | 100 | 1.0 | - | - | - |
| No.15 | 2.20 | 0.4 | 100 | 0.088 | 20 | 11.8 | 35.5 | 191.9 | 24.8 |
| Purified plasma | 1.56 | 0.128 | 100 | 0.02 | 100 | - | - | - | - |
| Frac.II | 21.84 | 1 | 100 | 2.18 | 100 | - | - | - | 1.0 |
| Purified No.14 | 1.26 | 0.158 | 100 | 0.02 | 40 | 52.0 | 156.0 | 844.5 | 109.0 |

| | | | | | | | | | |
|---|---|---|---|---|---|---|---|---|---|
| * The (IgG+IgM) content was used as an IG content. | | | | | | | | | |

Since 100µl of plasma and 100µl of No. 15 serum diluted to 1/2.5 times contain 1040µg and 88µg of IG(IgG+IgM), respectively, the titer had 11.8 times or more difference based on the ratio to plasma. For 300µl of plasma, the neutralizing activity could not be observed, suggesting that there was a 35 times or more difference. Compared with the group of mice, to which 300µl of Venilon was administered, the difference was 192 times because it contained 16.89mg/IG. In addition, concerning purified serum No. 14 (20µg/100µl), the difference was 109 times between Frac. II (2.18mg/100µl). The differences were 156 times compared with the 300µl of plasma and 845 times compared with the 300µl of Venilon. As mentioned above, it was demonstrated that antiserum obtained by immunizing the human antibody producing animals had better characters than those of human plasma.

### 5) Cross-reactivity in agglutination

While the IT-3 strain has a type B serotype, it is known that *Pseudomonas aeruginosa* has a plurality of serotypes (it is classified into 14 kinds of ○ immunogens by the *Pseudomonas aeruginosa* working group: Today's meaning of *Pseudomonas aeruginosa*, edited by Atsushi SAITO, Iyaku Journal, p.76, 1996). The cross-reactivity of the IT-3 strain immune serum to various types of *Pseudomonas* *aeruginosawas* was examined by agglutination method. In addition to the IT-3 strain, 122(A),IT-2(E),IT-1(G), and IT-4(I) (S.Sawadaet al, J. Gen. Microbiol., vol.133, p.3581, 1987), 97(B)strain (T. Kamimura, Arznein-Forsch./Drug Res. vol.34, p.1528, 1984), and the *Escherichia coli* 81 strain as a negative control(EC81:Tomioka et al., Clinical and Study, volume 55, p.3722, 1978) were cultured on the Tripticase Soy Agar(BBL) at 37°C over night, collected and suspended in the PBS. Then, the suspension was added with hormalin up to 1%, inactivated for a time period of 24 hours or more and used in the experiment. As the result from the examination of the No. 10 mouse serum, as shown in FIG.3, the antibody reacted only to the type B serotype of *Pseudomonas aeruginosawas*. This suggested that the immune serum obtained by administering only one serotype *Pseudomonas aeruginosawas* is thought to be specific to the serotype. As described above, it is known that MoAb against *Pseudomonas aeruginosawas* is specific to the serotype. It has been proven that the antiserum, which was separately immunized to the human antibody producing animal, also has similar characters to MoAb.

### Example 3 Immunization with a plurality of serotypes of Pseudomonas aeruginosawas

### 1) Immunogens

As immunogens, 1% hormalin inactivated strains 122, IT-3, IT-2, IT-1,and IT-4 were cultivated on the Trypticase Soy Agar (BBL) at 37°C over night, and collected and suspended in the PBS. Then, the suspension was added with hormalin up to 1%, inactivated for a time period of 24 hours or more and used in the experiment. The group of mouse immunized with the mixture of five kinds of 20µg of individual strains in an dose of 100µg/mouse in total with Freund's incomplete adjuvant at an interval of two weeks four times (mixed immunization group:No.41,42) and the group of mouse separately immunized with five kinds of strains in a dose of 100µg/mouse in total with an incomplete adjuvant at an interval of one week sequentially (sequential immunization group:No.43,44) were set.

### 2) ELISA antibody titers

The individual *Pseudomonas aeruginosa* of 122,IT-3,IT-2,IT-1,IT-4, IT-5(B), IT-6(C), IT-7(H) (S.Sawada et al, J. Gen. Microbiol., vol.133, p.3581, 1987),and IFO3080(M) (S.Sawada et al, J. Infec. Dis., vol.152, p.1290, 1985), which were used in immunizing TC mouse to obtain immune sera of both TC mice, were cultivated on Trypticase Soy Agar (BBL) at 37°C over night, collected and suspended in the PBS. Then, the suspension was added with hormalin up to 1%, inactivated for a time period of 24 hours or more and used. One % of hormalin inactivated *Pseudomonas aeruginosa* was solidified on the ELISA plate and similarly to the example 2, ELISA was performed to calculate the ELISA antibody titer/IG. The result is shown as the ratio of the antibody titer to that of plasma in Table 4.

**<Table 4>**

| Comparison of the ELISA antibody titer against *Pseudomonas aeruginosa* | | | | | | |
|---|---|---|---|---|---|---|
| *Pseudomonas aeruginosa* | TC mouse | | | | | Plasma |
| | Mixed immunization | | Sequential immunization | | IT-3 immunization | |
| | 41 | 42 | 43 | 44 | 11 | |
| 122(A) | 20.8 | 6.1 | 19.2 | 14.7 | 3.0 | 1 |
| IT-1(G) | 227.2 | 5.0 | 24.7 | 36.3 | 8.1 | 1 |
| IT-2(E) | 125.5 | 1.1 | 29.1 | 54.6 | 11.6 | 1 |
| IT-3(B) | 76.4 | 8.8 | 6.7 | 17.4 | 8.2 | 1 |
| IT-4(I) | 651.6 | 15.2 | 1.9 | 2.0 | 2.0 | 1 |
| IT-5(B) | 385.0 | 19.1 | 16.2 | 31.0 | 9.7 | 1 |
| IT-6(C) | 163.3 | 2.8 | 15.8 | 31.6 | 5.9 | 1 |
| IT-7(H) | 155.6 | 1.8 | 13.4 | 21.7 | 4.8 | 1 |
| IF03080(M) | 43.8 | 9.9 | 26.2 | 20.2 | 5.2 | 1 |

The immune serum obtained by either the mixed immunization or the sequential immunization group had cross-reactivity to all the *Pseudomonas aeruginosa* which was higher than that of the plasma. Comparing between the mixed immunization and sequential immunization group, the former had a significant higher antibody titer (No.41). In addition, to examine cross-reactivity of serotype of *Pseudomonas aeruginosa* that was not used for immunization, the antibody titer against *Pseudomonas aeruginosa* of IT-5(B), IT-6(C), IT-7(H), and IFO3080(M) was observed. As shown in Table 4, the result showed that the immune sera of both groups had higher ELISA antibody titer against all the immunized *Pseudomonas aeruginosa* and the non-immunized *Pseudomonas aeruginosa* than the plasma as well as having a higher cross-reactivity than the plasma. Moreover, they had higher ELISA antibody titer than that of the serum from the mouse immunized with only IT-3(B) strain (No.11).

### 3) Agglutination titer

The cross-reactivity to various kinds of *Pseudomonas aeruginos* was examined by agglutination. One % of hormalin inactivated *Pseudomonas aeruginos,* IT-5(B), IT-6(C), IT-7(H), and IFO3080(M), which were not used for immunization were used to examine the cross-reactivity by agglutination. The result was shown in FIG.4. The TC mouse immune sera of both of the mixed immunization group and sequential immunization group (No.41,42 and No.43,44, respectively) showed reactivity higher than that of the plasma and TC mouse immune serum obtained by immunizing only IT-3 (No.11) although.the reactivity varied among the groups. This means that the immune sera obtained by these methods has effects on a plurality of immunogens.

To examine whether this phenomenon is specific to the non-human animal having a human antibody gene locus, the mixed immunization and sequential immunization were performed to C57/BL mice, one kind of background mouse of the TC mouse under the same condition as that of the TC mouse and the cross-reactivity in the agglutination of the mouse serum was examined. As shown in FIG. 5, the serotype (in this case, IT-6(C)), to which the serum derived from the C57/BL mouse does not react, was present and showed the different cross-reactivity than that of the serum derived from the non-human animal having a human antibody gene locus. This means that the polyclonal antibody with a superior cross-reactivity can be prepared using the antibody gene locus of human rather than that of inherently existed in the animal.

From the fact that the polyclonal antibody having a superior properties to the polyclonal antibody produced from the normal animal immunized with an immunogen could be obtained, it may be considered that a difference between both of polyclonal antibodies is due to a difference in individual gene segments consisting of the region V, which forms the region which is bound to immunogens, between an animal and a human. This means that it is important to provide a group of gene segments composing the human antibody gene locus, especially the human antibody region V. This suggests that in case where the polyclonal antibody against a bacterium having a plurality of serotypes is produced in the non-human animal having a human antibody gene locus, by immunizing with, for example *Pseudomonas aeruginos* of at least two kinds out of several tens kinds of serotypes, the polyclonal antibody covering a wide spectrum of serotypes can be prepared.

### Example 4 Producing an anti-megalovirus antibody

### 1) Immunization with a cytomegalovirus

For *Cytomegalovirus* (CMV), an AD169 strain (ATCC No. VR-538) was used. The precipitation obtained by step-gradient centrifugation of the supernatant of the cultured Vero cells (ATCC No. CCL-81) infected by the AD169 strain with 45% (W/W) of glycerol (using a HITACHI RP42 rotor at 30,000rpm for 2hours) is suspended in the PBS to be prepared as virus particles. The precipitation, 50µl(1.2×10⁶PFU), was used for immunization for one mouse. The immunization was performed by intraperitoneally administering the AD169 strain to the TC mouse at a dose of 1.2×10⁶PFU/mouse, initially with a Freud's complete adjuvant and then, with an incomplete adjuvant at an interval of two weeks four times in total.

### 2)ELISA antibody titer

The CMV AD169 strain was solidified on ELISA plate by using the solution of the CMV AD169 strain having the concentration of 1.37×10⁶PFU/ml at 200µl/well and the ELISA antibody titer of the obtained CMV immunized TC mouse sera (No.12,13) were measured in the same way as that of the example 2. As shown in FIG.6 and Table 5, the antibody titer of TC mouse sera obtained from the mice (No.12,13) which was immunized with only CMV, was 0.9 to 1.3 times that of the plasma.

**<Table 5>**

| The ELISA antibody titer against CMV | | | |
|---|---|---|---|
| Sample | Immunogen | IgG content (µg/ml) for OD = 0.5 | Ratio to plasma |
| Plasma | - | 2.01 | 1.0 |
| No.12 | CMV | 2.15 | 0.9 |
| No.13 | CMV | 1.55 | 1.3 |

Generally, the CMV infection ratio of the Japanese is high and it is said that most of Japanese have been infected by CMV and produce an anti-CMV antibody(Latest Internal Medicine Taikei, volume 26, Viral Infections, edited by Hiroo IMURA et al., Makayama Shoten, p.147,1994). For this reason, it is known that the antibody titer of the IVIG prepared from such pool plasma has also high. It has been proved that the antibody titer of plasma used as a control against CMV is far higher than that of the human who has never infected by CMV and the serum having an antibody titer equal to or higher than that of the plasma could be obtained.

### Example 5 Producing an anti- Japanese encephalitis virus antibody

### 1) Immunization with a Japanese encephalitis virus

The immunogen of the *Japanese encephalitis* virus was prepared by infecting Vero cells(ATCC No. CCL-81) by a vaccine strain (Peking strain: Chemo-Sero-Therapeutic Research Institute ), cultivating the Vero cells for five days, inactivating the supernatant of the cultivated cells with 0.05% of hormalin, and performing sucrose density gradient centrifugation (at 24,000rpm, for 3 hours) two times.

The immunization was performed by intraperitoneally administering the immunogen to the TC mice initially with a Freud's complete adjuvant and then, with an incomplete adjuvant at an interval of two weeks four times in total. The group of mouse immunized with the immunogen of *Japanese encephalitis* virus at a dose of 5µg/mouse (No.2,3)and the group of mouse immunized with the mixture of the immunogen of *Japanese encephalitis* virus and 1% of hormalin inactivated *Pneumococcus* DIII5A strain (see the example 5: 100µg/mouse) was immunized(No.4, 5) were set.

### 2)ELISA antibody titer

Using the ELISA plate, on which 1µg/ml of inactivated and purified immunogen of *Japanese encephalitis* virus was solidified, the antibody titer of TC mouse immune serum (No.2,3,4,5) was measured in the same way as that of the example 2. As a result, as shown in FIG.7 and Table 6, the group of mouse immunized with only *Japanese encephalitis* virus showed the values 884.3 to 1079.1 times the plasma.

**<Table 6>**

| The ELISA antibody titers against *Japanese encephalitis virus* | | | |
|---|---|---|---|
| Sample | Immunogen | IgG content (µg/ml) for OD = 0.1 | Ratio to plasma |
| Plasma | - | 26.50 | 1.0 |
| No.2 | JEV* | 0.02 | 1079.1 |
| No.3 | JEV* | 0.03 | 884.3 |
| No.4 | JEV*&SP | 0.07 | 368.1 |
| No.5 | JEV*&SP | 1.07 | 24.8 |

| | | | |
|---|---|---|---|
| * JEV: *Japanese encephalitis virus* | | | |

On the other hand, the group of mouse immunized with the mixture with the *Pneumococcus* DIII5A strain showed far higher values, 24.8 to 368.1 times the plasma. In any case, the antibody with a considerably high titer could be obtained, which demonstrated that the antibody obtained from the human antibody production animals was far effective antibody compared to the plasma.

### 3)Neutralizing antibody titer

The neutralizing antibody titer of the obtained immune serum against the *Japanese encephalitis* virus was measured. The TC mouse sera (No.2,3) inactivated by being heated at 56°C for 30 minutes were gradually diluted and mixed with the same amount(2ml) of *Japanese encephalitis* virus (Peking strain) adjusted to the concentration of 2000 PFU/ml, incubated at 37°C for 90 minutes, and the Vero cells were applied on the 6-well plate at 2×10⁵cell/well, cultivated at 37°C under a condition of 5%CO₂ for two days. After the supernatant of wells was removed, 1% of methylcelrose MEM medium was added at 3 ml/well, and cultivated at 37°C under a condition of 5%CO₂ for 4 to 6 days until plaques developed. Ten % of hormalin was added on the plate at 1.5 ml/well and then stained with methylene blue to count the number of plaques. The reduction rate in plaque was calculated from the number of the plaques of the control well(medium was added) and plaques of individual diluted serum and the dilution factor at the point of 50% reduction in plaque was used as the neutralizing antibody titer. The result is shown in Table 7.

**<Table 7>**

| *in vitro* neutralizing antibody titer of *Japanese encephalitis virus* immunized TC mouse serum | | | | | | | | | | |
|---|---|---|---|---|---|---|---|---|---|---|
| Specimen/ dilution rate | Reduction rate of plaque | | | | | | Neutralizing antibody titer* | IG content (IgG+ IgM)** | Neutralizing antibody titer/IG content | Ratio to plasma |
| | 10 | 40 | 160 | 640 | 2560 | 10240 | | | | |
| Plasma | 92.3 | 29.5 | -6.7 | - | - | - | 29.1 | 10.4 | 2.8 | 1.0 |
| No.2 | 97.0 | 87.2 | 80.2 | 63.1 | 36.6 | 17.4 | 971.2 | 4.3 | 224.8 | 80.3 |
| No.3 | 94.6 | 59.7 | 21.8 | 15.1 | 6.4 | - | 88.8 | 2.3 | 38.9 | 13.9 |

| | | | | | | | | | | |
|---|---|---|---|---|---|---|---|---|---|---|
| * Neutralizing antibody titer calculated by 50% plaque reduction | | | | | | | | | | |
| ** mg/ml | | | | | | | | | | |

The neutralizing antibody titer of the plasma was 29.1 and those of the immunized TC mice(No.2,3) were 971.2 and 88.8, respectively. Comparing among them by converting to the values equivalent to IG(IgG+IgM) content, the immunized TC mouse sera had higher neutralizing antibody titers, 13.9 to 80.3 times that of the plasma. Thus, it was proven that the antibody obtained according to the present invention had a high usefulness.

### Example 6 Producing an anti-Pneumococcus antibody

### 1) Immunization with a Pneumococcus virus

For *Pneumococcus* virus , the DIII5A strain (Tomiokac et al., Clinical and Study, volume 55, p.3722,1978) was cultivated on the Trypticase Soy II, 5% of sheep blood agar medium(BBL) at 37°C over night, collected and suspended in the PBS. Then the suspension was added with hormalin up to 1%, and inactivated for a time period of 24 hours or more and then used. The immunization was performed by intraperitoneally administering the immunogen to TC mouse initially with a Freud's complete adjuvant and then, with an incomplete adjuvant at an interval of two weeks for times in total. The group of mouse immunizaed with the DIII5A strain at 100µg/mouse (No.1) and the group of mouse immunized with the mixture of the DIII5A strain (100µg/mouse) and the immunogen of the *Japanese encephalitis* virus(see the example 5: 5µg/mouse) (No.4,5) were set.

### 2)ELISA antibody titer

Using the plate on which 1% of hormalin inactivated Pneumococcus virus was solidified, the antibody titer of the immunized TC mouse sera (No.1,4,5) were measured in the same way as that of the example 2 and compared between the values of these groups. As shown in Figure 8 and Table 8, both of the sequential immunization (No.1) and mixed immunization (No.4,5)group showed the equal or higher level of antibody titer than that of the plasma, 0.8 to 1.6 times them.

**<Table 8>**

| The ELISA antibody titer against *Pneumococcus (SP)* | | | |
|---|---|---|---|
| Sample | Immunogen | IgG content (µg/ml) for OD = 0.05 | Ratio to plasma |
| Plasma | - | 2.60 | 1.0 |
| No.1 | SP | 3.15 | 0.8 |
| No.4 | JEV*&SP | 1.62 | 1.6 |
| No.5 | JEV*&SP | 2.39 | 1.1 |

| | | | |
|---|---|---|---|
| * JEV: *Japanese encephalitis virus* | | | |

### Example 7 Producing an anti E.coli antibody

### 1) Immunization with an E. coli

*E.coli* 81 strain(Tomiokaet al., Clinical and Study, volume 55, p.3722, 1978) was cultivated on the Trypticase Soy Agar (BBL) at 37°C over night, collected and suspended in the PBS. Then, the suspension was added with hormalin up to 1%, inactivated for a time period of 24 hours or more, and then used for as an immunogen. The immunization was performed by intrapreritoneally administering the TC mouse with the immunogen at 100 µg/mouse initially with a Freud's complete adjuvant and then, with an incomplete adjuvant at an interval of two weeks four times in total (No.31, 32).

### 2) Agglutination titer

The agglutination titer of the *E.coli* 81 strain immunized TC mouse sera (No.31, 32) was measured. One % of hormalin inactivated E.coli 81 strain was diluted to obtain a solution of 2mg/ml with a 1% BSA, 50mM Tris, 0.15M NaCl solution (pH8.0), and the agglutination titer was measured in the same way of that of the example 2. As shown in FIG.9, up to 1/40 concentration, agglutination titer was observed in TC mouse sera, while in the plasma group, no agglutination titer was observed even though the sample solution was diluted to 1/2 concentration.

Considering the difference in IG content between the No.31 mouse serum and the plasma(2.16 mg/ml and 10.40 mg/ml), at least a major difference exists by a factor of 96.4.

### Example 8 Producing an anti-MRSA antibody

### 1) Immunization with an MRSA

MRSA(provided by Chemo-Sero-Therapeutic Research Institute) was cultivated on the Trypticase Soy Agar (BBL) at 37°C over night, collected and suspended in the PBS. Then, the suspension was added with hormaline up to 1%, inactivated for a time period of 24 hours and more, and then used for as an immunogen. The immunization was performed by intrapropeneally administering the immunogen to the TC mouse at 100 µg/mouse initially with a Freud's complete adjuvant and then, with an incomplete adjuvant at an interval of two weeks four times in total(No.33, 34).

### 2) Agglutination titer

The agglutination titer of the MRSA immunized TC mouse (No.33, 34) sera were measured. One % of hormalin inactivated MRSA was diluted with a 1% BSA, 50 mM Tris, 0.15 M NaCl solution (pH8.0) to obtain a 2 mg/ml of solution and the agglutination titer was measured in the same way as that of the example 1. As shown in FIG. 10, in the TC mouse sera, agglutination was observed up to 1/1280 to 1/2560 concentration. On the other hand, in the plasma, agglutination was observed up to 1/2048 concentration. The IG content contained in the plasma (IgG+IgM) was 10.40 mg/m, and for example, the IG content of the TC mouse No.34 was 1.48 mg/ml, thereby the value of the TC mouse immunogenic serum showed 4.4 times that of the plasma.

### INDUSTRIAL APPLICABILITY

The present invention provides a drug for preventing or treating the infections in which pathogens such as bacteria and viruses, especially the infections developed in the post-operative patients and the severe patients with underlying diseases such as cancers, the elder, infant and fatal patients (compromised hosts), whose immunological competence was compromised, the infections on which no antibiotic drug has effects, and emerging infections for which no treatment has been found.

The contents of all the publications stated in the specification are included in the specification. In addition, those skilled in the art may be easy to understand that the present invention may be altered and modified in the various way within the technical scope and the range of the present invention. The present invention intends to include these alternations and modifications in the present invention.

## Claims

1. A human polyclonal antibody composition for preventing or treating infections, comprising a human polyclonal antibody obtained by administering a bacterium and/or a virus or a component of the bacterium and/or the virus as immunogen to a non-human animal having a human antibody gene locus, wherein the human antibody polyclonal antibody compositions having a higher antibody titer against the bacterium and/or the virus than that of human pool plasma.

2. The human polyclonal antibody composition defined in claim 1, wherein they have at least 1.1 times or more ELISA antibody titer against the bacterium and/or the virus used in administering, 4.4 times or more agglutination titer, and at least 14 times or more neutralizing antibody titer compared with that of human pool plasma.

3. The human polyclonal antibody composition defined in claim 1 or 2, further comprising a polyclonal antibody, which recognizes the bacteria and/or viruses other than the bacteria and/or the viruses administered as immunogen.

4. The human polyclonal antibody composition defined in any of claims 1 to 3, which can be obtained by administering at least two kinds of the bacterium and/or virus or the components of the bacterium and/or the virus and react to at least these two kinds of bacteria and/or viruses.

5. The human polyclonal antibody compositionsdefined in any of claims 1 to 4, of which immunogen is selected from a gram positive bacteria or a gram negative bacteria.

6. The human polyclonal antibody composition defined in any of claims 1 to 5 of which immunogen is selected from a group of Pseudomonas aeruginosa, Pneumococcus, E.coli, and Staphylococcus aureus.

7. The polyclonal antibody composition define in any of claims 1 to 6, of which immunogen is a drug resistance drug.

8. The human polyclonal antibody composition defined in claim 7, containing as a drug resistance bacterium MRSA.

9. The human polyclonal antibody composition define in any of claims 1 to 4 which can be obtained using an immunogen selected from DNA viruses or RNA viruses.

10. The polyclonal antibody composition defined in claim 9, of which immunogen is selected from cytomegalovirus or Japanese encephalitis virus.

11. The human polyclonal antibody composition defined in any of claims 1 to 10, wherein a non-human animal having a human antibody gene locus is an animal having a plurality of human region V gene segments.

12. The human polyclonal antibody composition defined in claim 11, wherein the plurality of human region V gene segments are eight or more human V region gene segments.

13. The human polyclonal antibody which can be obtained by administering as immunogen a bacterium and/or a virus or a component of the bacterium and/or the virus and recognizes the bacteria and/or the viruses administered as immunogen other than bacteria and/or the viruses administered as immunogen.

14. The polyclonal antibody define in claim 13, which can be obtained by administering at least of two kinds of bacteria and/or viruses or the components of the bacteria/ and or the viruses.

15. The human polyclonal antibody wherein serotype of bacteria and/or viruses obtained by administering at least two kinds of bacteria and/or viruses or the components of the bacteria and the viruses are used as immunogen and recognize the serotype of bacteria and/or the viruses administered as immunogen and other bacteria and/or viruses.

16. The polyclonal antibody def ined in claim 15, wherein the bacterium is *Pseudomonas aeruginosa.*

17. The human polyclonal antibody which can be obtained by administering as immunogen serotypes of *Pseudomonas aeruginos* 122, IT-1, IT-2, IT-3, and IT-4 to the non-human animal having the human antibody gene locus and has reactivity against serotypes of *Pseudomonas aeruginos* 122, IT-1, IT-2, IT-3, IT-4, IT-5, IT-6, IT-7, and IFO3080.

18. The human polyclonal antibody defined in any of claims 13 to 17, wherein the non-human animal having the human antibody gene locus is a animal having a plurality of region V gene segments.

19. The human polyclonal antibody defined in claim 18, wherein the number of a plurality of human V region gene segments are right or more VH and VL region segments in total.

20. The human polyclonal antibody composition defined in any of claims 13 to 19, for preventing or treating infections including the human polyclonal antibody.

21. The human polyclonal antibody composition defined in any of claims 1 to 13, which is used as substitution for an immunoglobulin drug.

22. A method for preparing the human polyclonal antibody defined in claim 1, comprising a step for administering using a bacterium and/or a virus or a component of the bacterium and/or the virus to the non-human animal having the human antibody gene locus.

23. The method for preparing the human polyclonal antibody defined in any of claims 1 to 13 and 20, comprising a step for administering as immunogen a bacterium and/or a virus or a component of the bacterium and/or the virus to the non-human animal having the human antibody gene locus.

24. The human polyclonal antibody composition for preventing or treating infections by bacteria and/or viruses, which has a ELISA antibody titer against the bacterium and/or the virus is at least 1.1times or more, agglutination titer is at least 4.4 times or more, and neutralizing antibody titer is at least 14 times or more compared with those of human pool plasma.

25. The human polyclonal antibody composition defined in claim 24 , wherein the bacterium is *Pseudomonas aeruginosa*.

26. The human polyclonal antibody composition defined in claim 25, containing *Pseudomonas aeruginosa* of two or more kinds of serotypes.

27. The human polyclonal antibody composition defined in claim 26, wherein *Pseudomonas aeruginosa* is serotypes 122,IT-1,IT-2,IT-3,IT-4,IT-5,IT-6,IT-7, and IF03080.

28. The human polyclonal antibody composition defined in any of claims 24 to 27, which is used as a substitution for an immunoglobulin drug.
